(19) Europäisches Patentamt
European Patent Office
Office européen des brevets

(11) Veröffentlichungsnummer: **0 243 921**
**A2**

(12) # EUROPÄISCHE PATENTANMELDUNG

(21) Anmeldenummer: **87106095.0**

(22) Anmeldetag: **27.04.87**

(51) Int.Cl.[3]: **C 07 D 499/00**
A 61 K 31/43

(30) Priorität: **29.04.86 CH 1747/86**

(43) Veröffentlichungstag der Anmeldung:
**04.11.87 Patentblatt 87/45**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Hirt, Hans, Dr.**
**Jupiterstrasse 20**
**CH-4123 Allschwil(CH)**

(72) Erfinder: **Eugster, Hansjörg**
**Aeschistrasse 37**
**CH-3362 Niederönz(CH)**

(74) Vertreter: **Zumstein, Fritz, Dr. et al,**
**Bräuhausstrasse 4**
**D-8000 München 2(DE)**

(54) **Kristallines Hydrat eines Penem-Derivats.**

(57) Die Erfindung betrifft das kristalline Monohydrat der (5R,6S)-2-Aminomethyl-6-[(1R)-1-hydroxyäthyl]-2-penem-3-carbonsäure und Verfahren zur Herstellung desselben. Die Substanz ist zur Behandlung von Infektionserkrankungen geeignet.

EP 0 243 921 A2

Croydon Printing Company Ltd.

0243921

CIBA-GEIGY AG
Basel (Schweiz)

4-15857/+

Kristallines Hydrat

Die Erfindung betrifft das kristalline Monohydrat der (5R,6S)-2-Aminomethyl-6-[(1R)-1-hydroxyäthyl]-2-penem-3-carbonsäure und Verfahren zur Herstellung desselben.

In der Deutschen Offenlegungsschrift Nr. 3431980 sind die (5R,6S)-2-Aminomethyl-6-[(1R)-1-hydroxyäthyl]-2-penem-3-carbonsäure, Verfahren zu ihrer Herstellung und ihre Verwendung als antibakterielles Mittel beschrieben. Die Verbindung weist ein ausserordentlich breites antibakterielles Wirkungsspektrum auf, das die bekannten grampositiven und gramnegativen Erreger, einschliesslich Problemkeime, wie Methicillin-resistente Staphylokokken und Pseudomonas aeruginosa, umfasst, und kann dementsprechend als parenteral applizierbares Breitspektrum-Antibiotikum verwendet werden. Nach dem in der genannten Deutschen Offenlegungsschrift beschriebenen Verfahren fällt die Verbindung in amorpher Form an.

Amorphe Substanzen sind mit verschiedenen Nachteilen behaftet, die sie zur Verwendung, insbesondere zur Herstellung von pharmazeutischen Präparaten, als wenig geeignet oder sogar ungeeignet erscheinen lassen. Ein Nachteil ist in der, verglichen mit kristallisierten Substanzen, relativ grossen Oberfläche der amorphen Substanzen zu sehen, die, verbunden mit der regellosen thermodynamisch ungünstigen Anordnung der Moleküle im Festkörper, für eine beträchtlich grössere Anfälligkeit gegenüber äusseren Einflüssen, wie Luftsauerstoff, Licht und erhöhte Temperatur, verantwortlich ist. Weiterhin neigen amorphe Substanzen in weit grösserem Ausmass

als kristallisierte Verbindungen dazu, Lösungsmittel einzuschliessen und sich der Abgabe dieser Verunreinigungen, beispielsweise bei der Trocknung, hartnäckig zu widersetzen. Derartige Verunreinigungen, insbesondere schädliche Lösungsmittel, wie Acetonitril oder chlorierte Kohlenwasserstoffe, enthaltende Präparate sind für die medizinische Verwendung, insbesondere bei parenteraler Applikation, nicht geeignet. Ein weiterer Nachteil amorpher Produkte liegt darin, dass Luftfeuchtigkeit in erheblich grösserem Masse, als dies bei kristallinen Produkten der Fall ist, aufgenommen wird. Der steigende Wassergehalt solcher Produkte erschwert einerseits die Herstellung von pharmazeutischen Präparaten mit einem konstanten Wirkstoffgehalt und hat darüberhinaus einen negativen Einfluss auf die Rieselfähigkeit des Produktes. Amorphe Produkte besitzen ein relativ grosses Schüttvolumen, was die Verwendung grösserer Gefässe bei der Lagerung und bei der Herstellung von Arzneimittelzubereitungen erforderlich machen kann. Das oft mangelhafte Lösungsverhalten von amorphen Produkten (diese "verpappen" oder verkleben leicht, wodurch sich die Auflösungsgeschwindigkeit verringert) soll in diesem Zusammenhang ebenfalls erwähnt werden.

Die genannten Nachteile von amorphen Produkten, insbesondere die geringe Lagerstabilität, lassen es wünschenswert erscheinen, eine von der amorphen Form verschiedene Aggregatform aufzufinden, welche die für einen Arzneiwirkstoff zu fordernden Eigenschaften, wie insbesondere gute Lagerstabilität, aufweist.

Es wurde nun gefunden, dass das kristalline Monohydrat der (5R,6S)-2-Aminomethyl-6-[(1R)-1-hydroxyäthyl]-2-penem-3-carbonsäure die an einen Arzneimittelwirkstoff gestellten Anforderungen, insbesondere hinsichtlich Lagerstabilität und Verarbeitbarkeit, in hervorragender Weise erfüllt.

1 Mol des Kristallisats enthält konstant 1 Mol Wasser. Es handelt sich daher um ein definiertes Monohydrat. Unter der Bezeichnung "kristallin" ist zu verstehen, dass das Produkt im wesentlichen frei von amorphen Bestandteilen ist.

Insbesondere betrifft die Erfindung das kristalline Monohydrat der (5R,6S)-2-Aminomethyl-6-[(1R)-1-hydroxyäthyl]-2-penem-3-carbonsäure, gekennzeichnet durch die nachfolgenden Gitterabstände (d-Werte) über 2,8 Ångström und relativen Linienintensitäten ihres Röntgenpulverdiagramms (Kamera nach Guinier, Strahlungsquelle Kupfer-$K_{\alpha1}$):

| d-Werte (Ångström) | relative Intensität |
|---|---|
| 8.3 | mittel |
| 6.6 | sehr stark |
| 5.46 | stark |
| 4.97 | stark |
| 4.75 | sehr stark |
| 4.54 | stark |
| 4.19 | mittel |
| 4.12 | schwach |
| 3.85 | sehr stark |
| 3.79 | stark |
| 3.67 | schwach |
| 3.58 | stark |
| 3.40 | stark |
| 3.33 | sehr schwach |
| 3.28 | schwach |
| 3.27 | mittel |
| 3.24 | mittel |
| 3.17 | sehr stark |
| 3.13 | sehr schwach |
| 2.99 | sehr stark |
| 2.98 | mittel |
| 2.93 | sehr stark |
| 2.88 | schwach |

Das Produkt weist eine sehr gute Kristallinität auf, ist gut filtrierbar, ist auch bei längerer Einwirkung von Licht, Wärme (40°C) und Luftsauerstoff sehr stabil und zeigt bei annähernd

normalen Umgebungsbedingungen keine Tendenz, grössere Mengen von Wasser aus der Luft aufzunehmen. Die Lagerstabilität ist somit als gut zu bezeichnen. Im Vergleich zu der vorbekannten amorphen 2-Aminomethyl-6-[(1R)-1-hydroxyäthyl]-2-penem-3-carbonsäure weist das erfindungsgemässe Produkt einen höheren Reinheitsgrad auf und braucht darüberhinaus zur Abtrennung von Lösungsmittelresten nicht lyophilisiert zu werden, sondern kann nach gewöhnlichem Trocknen im Vakuum weiterverarbeitet werden. Das Produkt ist gut fliessfähig, lässt sich leicht maschinell abfüllen und kann in der gewünschten Menge ohne Schwierigkeiten zu lagerstabilen parenteral applizierbaren Arzneimittelzubereitungen verarbeitet werden.

Die Erfindung betrifft ebenfalls ein Verfahren zur Herstellung des kristallinen Monohydrates der (5R,6S)-2-Aminomethyl-6-[(1R)-1-hydroxyäthyl]-2-penem-3-carbonsäure. Ueberraschenderweise wurde gefunden, dass man das kristalline Monohydrat erhalten kann, indem man eine übersättigte Lösung eines (5R,6S)-2-Aminomethyl-6-[(1R)-1-hydroxyäthyl]-2-penem-3-carbonsäure-Monohydrat-Aethersolvats in einem wasserhaltigen $C_1$-$C_4$-Alkanol herstellt, oder ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, die Lösung des gebildeten Sulfonat-Salzes, gegebenenfalls nach vorhergehender Isolierung des Sulfonats aus dem Reaktionsgemisch und anschliessender Wiederauflösung des Isolats in Wasser oder in einem Gemisch bestehend aus Wasser und einem $C_1$-$C_4$-Alkanol, durch Zugabe äquimolarer Mengen einer organischen Stickstoffbase und eines $C_1$-$C_4$-Alkanols in eine übersättigte Lösung der freien Penem-Verbindung überführt, das Produkt zur Kristallisation bringt, und die Kristalle isoliert und trocknet.

Unter einem (5R,6S)-2-Aminomethyl-6-[(1R)-1-hydroxyäthyl]-2-penem-3-carbonsäure-Monohydrat-Aethersolvat ist insbesondere ein entsprechendes Aethersolvat mit einem wasserlöslichen niederaliphatischen oder 5- oder 6-gliedrigen cycloaliphatischen Aether zu verstehen. Solche Aether sind beispielsweise 1,2-Dimethoxyäthan,

Dioxan und Tetrahydrofuran. Bevorzugt als Ausgangsstoff ist das Monohydrat-Tetrahydrofuran-Hemisolvat der (5R,6S)-2-Aminomethyl-6-[(1R)-1-hydroxyäthyl]-2-penem-3-carbonsäure.

Unter einer Lösung eines (5R,6S)-2-Aminomethyl-6-[(1R)-1-hydroxyäthyl]-2-penem-3-carbonsäure-Monohydrat-Aethersolvats in einem wasserhaltigen $C_1$-$C_4$-Alkanol ist beispielsweise eine Lösung in einem Lösungsmittelgemisch, bestehend aus Wasser und Methanol, Wasser und Aethanol, Wasser und n-Propanol sowie Wasser und Isopropanol zu verstehen. Bevorzugt als Alkanol-Komponenten sind Isopropanol und insbesondere Aethanol. Der Wassergehalt eines derartigen wasserhaltigen $C_1$-$C_4$-Alkanols beträgt z.B. 5-30 Volumen-%, bevorzugt 9-20 Volumen-%. Eine übersättigte Lösung gemäss der ersten Verfahrensvariante kann hergestellt werden, indem man irgendeine Form des (5R,6S)-2-Aminomethyl-6-[(1R)-1-hydroxyäthyl]-2-penem-3-carbonsäure-Monohydrat-Aethersolvats, z.B. die amorphe Form oder die kristalline Form, in Wasser bei Raumtemperatur oder bevorzugt bei erhöhter Temperatur, z.B. bis zum Siedepunkt des verwendeten Lösungsmittels, bevorzugt aber bis maximal 50°C, unter Vermeidung oder Entfernung vorhandener Kristallisationskeime bis zur Sättigung löst und die erhaltene reine Lösung in den Zustand der Uebersättigung bringt. Dazu wird die gesättigte Lösung, die eine Temperatur von etwa 20° bis etwa 50°C, bevorzugt von etwa 40° bis etwa 50°C, aufweist, mit einem Ueberschuss des $C_1$-$C_4$-Alkanols, insbesondere mit einem 4-10fachen Ueberschuss des $C_1$-$C_4$-Alkanols bezogen auf die eingesetzte Wassermenge, versetzt und langsam abgekühlt, beispielsweise auf eine Temperatur zwischen 0° und etwa -30°C, bevorzugt auf etwa -25°C. Die Kristallbildung kann spontan erfolgen, beispielsweise an der Oberfläche des Reaktionsgefässes bzw. Rührgerätes, kann aber auch durch Animpfen, d.h. Einbringen von Impfkristallen, ausgelöst werden. Stehen keine Impfkristalle zur Verfügung, können diese, vorteilhaft in einem aliquoten Teil der Lösung, in üblicher Weise, beispielsweise durch heftiges Schütteln, Einbringen von Glasstaub oder Ritzen der Gefässwand, hergestellt werden. In einer bevorzugten Ausführungsform der ersten Verfahrensvariante wird die übersättigte Lösung mit Impfkristallen angeimpft. Vor Herstellung

der übersättigten Lösung können ein oder mehrere Reinigungsschritte, z.B. Aktivkohle-Behandlung und/oder Filtration, wie insbesondere Sterilfiltration, eingeschoben werden.

In einer zweiten Verfahrensvariante des erfindungsgemässen Verfahrens wird ausgehend vom (5R,6S)-2-Aminomethyl-6-[(1R)-1-hydroxyäthyl]-2-penem-3-carbonsäure-Monohydrat-Aethersolvat ein Sulfonat hergestellt, welches gegebenenfalls isoliert werden kann, und dieses wird in einem Folgeschritt in das erfindungsgemässe Monohydrat überführt. Gegenüber der oben beschriebenen ersten Verfahrensvariante bietet die "Sulfonat-Variante" den grossen Vorteil, dass das (5R,6S)-2-Aminomethyl-6-[(1R)-1-hydroxyäthyl]-2-penem-3-carbonsäure-Sulfonat eine sehr gute Wasserlöslichkeit aufweist, sodass die als Lösungsmittel erforderliche Menge Wasser signifikant gesenkt werden kann. Neben den sich daraus ergebenden verfahrensökonomischen Vorteilen (geringere Flüssigkeitsvolumina, kleiner dimensionierte Reaktionsgefässe, niedrigere Energiekosten beim Abkühlen zum Auskristallisieren des Endproduktes) tritt auch eine Ausbeutesteigerung ein, da durch die geringeren Lösungsmittelvolumina die Produktverluste in den Mutterlaugen zurückgehen. Darüberhinaus bietet die Möglichkeit, das intermediär gebildete Sulfonat zu isolieren, den Vorteil eines zusätzlichen Reinigungsschrittes. Das Verfahren ist daher zur Durchführung im industriellen Massstab geeignet.

Unter einer Sulfonsäure ist in erster Linie eine gegebenenfalls, z.B. durch Halogen, wie Fluor, substituierte $C_1$-$C_4$-Alkansulfonsäure, z.B. Methansulfonsäure, oder eine gegebenenfalls, z.B. durch $C_1$-$C_4$-Alkyl, wie Methyl, oder Halogen, wie Brom, substituierte Benzolsulfonsäure, z.B. Benzolsulfonsäure, p-Toluolsulfonsäure oder p-Brombenzolsulfonsäure, zu verstehen. Bevorzugt als Sulfonsäure ist Methansulfonsäure.

Eine organische Stickstoffbase ist insbesondere ein gegebenenfalls substituiertes niederaliphatisches primäres, sekundäres oder tertiäres Amin, wie insbesondere ein gegebenenfalls durch Hydroxy substituiertes Mono-, Di- oder Tri-$C_1$-$C_4$-alkylamin, z.B. Triäthyl-

amin, Diisopropylamin, Aethanolamin, Diäthanolamin oder Triäthanolamin, ein Cyclo-$C_5$-$C_6$-alkylamin, z.B. Cyclohexylamin, oder ein cyclisches Amin mit 4 oder 5 Kohlenstoffatomen, z.B. Pyrrolidin, Piperidin oder Morpholin. Bevorzugt als Stickstoffbase sind die genannten niederaliphatischen Amine, insbesondere Triäthylamin.

Für das Verfahren wird das Ausgangsmaterial, das (5R,6S)-2-Aminomethyl-6-[(1R)-1-hydroxyäthyl]-2-penem-3-carbonsäure-Monohydrat-Aethersolvat in einer geringen Menge Wasser oder in einem Gemisch bestehend aus Wasser und einem $C_1$-$C_4$-Alkanol, z.B. einem der oben genannten, insbesondere Aethanol oder Isopropanol, suspendiert. Es ist nicht erforderlich, eine Lösung herzustellen, da eine solche bei der nachfolgenden Bildung des Sulfonates eintritt. Bevorzugt erfolgt die Reaktion mit einer äquimolaren Menge der Sulfonsäure in Wasser oder in einem Gemisch bestehend aus Wasser und dem $C_1$-$C_4$-Alkanol mit einem Wassergehalt von ca. 25-75 %, bei Raumtemperatur oder geringfügig erniedrigter Temperatur, z.B. in einem Temperaturbereich von ca. 0° bis ca. 20°C. Aequimolare Mengen der organischen Stickstoffbase, inbesondere Triäthylamin, werden bei gleicher Temperatur in Form einer Lösung in dem gleichen $C_1$-$C_4$-Alkanol hinzugegeben, wobei nach erfolgter Zugabe das Gesamtvolumenverhältnis Wasser-$C_1$-$C_4$-Alkanol zur Erreichung des Uebersättigungszustandes in einem Bereich zwischen 1:3 und 1:10, bevorzugt bei etwa 1:3 bis etwa 1:5, liegen soll. Die übersättigte Lösung wird danach langsam auf eine Temperatur zwischen 0° und etwa -30°C, insbesondere auf etwa -25°C, abgekühlt. Die Kristallbildung kann spontan erfolgen oder kann durch Animpfen, z.B. wie oben beschrieben, ausgelöst werden.

Vorzugsweise werden vor Zugabe der organischen Stickstoffbase ein oder auch mehrere Reinigungsschritte eingeschoben. Beispielsweise kann die Sulfonat-Lösung einer Aktivkohle-Behandlung und/oder Filtration, wie insbesondere einer Sterilfiltration, unterzogen werden.

Zur Erzielung eines zusätzlichen Reinigungseffektes kann das intermediär gebildete Sulfonat aus dem Reaktionsgemisch isoliert werden. Ueberraschenderweise wurde gefunden, dass sich das Sulfonat in kristalliner Form erhalten lässt, wenn man die wässrige oder wässrig-$C_1$-$C_4$-alkanolische Lösung des Sulfonats in einem Temperaturbereich von ca. 0° bis etwa 20°C mit einem Ueberschuss des $C_1$-$C_4$-Alkanols bis zu einem Volumenverhältnis Wasser-$C_1$-$C_4$-Alkanol von ca. 1:3 bis 1:10, bevorzugt von etwa 1:5 bis etwa 1:8, versetzt. Das Sulfonat fällt in hochreiner kristalliner Form an, so dass Verunreinigungen mit der Mutterlauge problemlos abgetrennt werden können. Das kristalline Sulfonat wird in üblicher Weise abgetrennt, bei Raumtemperatur oder etwas erniedrigter Temperatur, z.B. bei etwa 0° bis etwa 20°C, in wenig Wasser oder in einem Gemisch bestehend aus Wasser und einem $C_1$-$C_4$-Alkanol gelöst und bei gleicher Temperatur mit äquimolaren Mengen der organischen Stickstoffbase, gelöst im gleichen $C_1$-$C_4$-Alkanol, versetzt, wobei nach erfolgter Basenzugabe das Gesamtvolumenverhältnis Wasser-$C_1$-$C_4$-Alkanol zur Erreichung des Uebersättigungszustandes in einem Bereich zwischen 1:3 und 1:10 liegen soll. Die übersättigte Lösung wird danach langsam auf eine Temperatur zwischen 0° und etwa -30°C, insbesondere auf etwa -25°C, abgekühlt. Die Kristallbildung kann spontan erfolgen oder kann durch Animpfen, z.B. wie oben beschrieben, ausgelöst werden.

Die gebildeten neuen kristallinen Verbindungen, insbesondere das (5R,6S)-2-Aminomethyl-6-[(1R)-1-hydroxyäthyl]-2-penem-3-carbonsäure-Monohydrat , können mit Hilfe beliebiger für die Trennung binärer fest/flüssiger Systeme zur Verfügung stehender Methoden isoliert und gesammelt werden, beispielsweise durch Filtration, Druckfiltration ("Abnutschen"), Zentrifugieren oder Dekantieren. Zur Entfernung von in verbleibenden Mutterlaugeresten enthaltenen Verunreinigungen kann mit dem zur Kristallisation verwendeten $C_1$-$C_4$-Alkanol, der kleine Mengen Wasser (ca. 2-10 %) enthält, nachgewaschen werden.

Die Trocknung wird bei normaler oder leicht erhöhter Temperatur, beispielsweise im Temperaturbereich von etwa 15°C bis etwa 40°C, vorzugsweise bei etwa 20° bis etwa 25°C (Raumtemperatur), durchgeführt und bis zu annähernder Gewichtskonstanz fortgesetzt. Zur Beschleunigung der Trocknung kann unter vermindertem Druck gearbeitet werden, wobei z.B. sogenanntes Wasserstrahlvakuum (etwa 650 bis etwa 3300 Pa) oder Hochvakuum (etwa 5 bis etwa 100 Pa) angewendet werden kann.

Die Erfindung betrifft weiterhin die als Zwischenprodukte erhältlichen Sulfonate der (5R,6S)-2-Aminomethyl-6-[(1R)-1-hydroxyäthyl]-2-penem-3-carbonsäure in amorpher und in kristalliner Form und Verfahren zu ihrer Herstellung.

Insbesondere betrifft die Erfindung das (5R,6S)-2-Aminomethyl-6-[(1R)-1-hydroxyäthyl]-2-penem-3-carbonsäure-Methansulfonat in amorpher und in kristalliner Form. Die kristalline Form des Methansulfonats ist durch die folgenden Gitterabstände (d-Werte) über 3,0 Ångström und relativen Linienintensitäten ihres Röntgenpulverdiagramms (Kamera nach Guinier, Strahlungsquellen Kupfer-$K_{\alpha 1}$) gekennzeichnet:

| d-Werte (Ångström) | relative Intensität |
|---|---|
| 15.0 | sehr stark |
| 13.1 | schwach |
| 10.7 | mittel |
| 9.2 | sehr stark |
| 8.8 | sehr schwach |
| 7.5 | schwach |
| 6.7 | schwach |
| 6.5 | mittel |
| 5.86 | sehr schwach |
| 5.32 | schwach |
| 4.97 | schwach |
| 4.86 | mittel |
| 4.77 | mittel |
| 4.61 | stark |
| 4.57 | schwach |
| 4.41 | mittel |

| d-Werte (Ångström) | relative Intensität |
|---|---|
| 4.18 | schwach |
| 4.09 | schwach |
| 4.01 | sehr schwach |
| 3.94 | stark |
| 3.69 | stark |
| 3.61 | mittel |
| 3.58 | mittel |
| 3.52 | schwach |
| 3.43 | mittel |
| 3.40 | schwach |
| 3.31 | stark |
| 3.24 | mittel |
| 3.18 | sehr schwach |
| 3.12 | sehr schwach |
| 3.06 | mittel |
| 3.02 | mittel |

Das Verfahren zur Herstellung der (5R,6S)-2-Aminomethyl-6-[(1R)-1-hydroxyäthyl]-2-penem-3-carbonsäure-Sulfonate ist dadurch gekennzeichnet, dass man ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, durch Zugabe eines Ueberschusses des $C_1$-$C_4$-Alkanols eine übersättigte Lösung herstellt, das Produkt zur Kristallisation bringt, und die Kristalle isoliert und trocknet.

Die verfahrensgemäss als Ausgangsstoffe verwendeten (5R,6S)-2-Aminomethyl-6-[(1R)-1-hydroxyäthyl]-2-penem-3-carbonsäure-Monohydrat-Aethersolvate sind neu und ebenfalls ein Gegenstand der vorliegenden Erfindung. Sie können hergestellt werden, indem man eine Verbindung der Formel

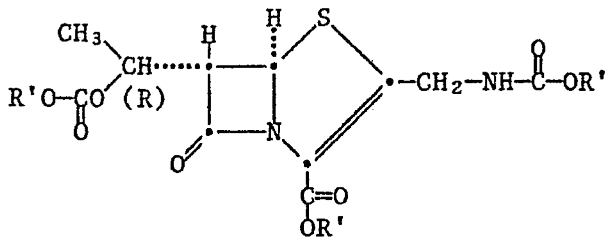

(I)

worin R' für gegebenenfalls substituiertes Allyl steht, in einem Gemisch bestehend aus einem wasserlöslichen niederaliphatischen oder 5- oder 6-gliedrigen cycloaliphatischen Aether mit einem Allylgruppenakzeptor in Gegenwart eines geeigneten Palladium(O)-Katalysators und gegebenenfalls in Gegenwart von Triphenylphosphin umsetzt.

Gegebenenfalls substituiertes Allyl ist beispielsweise Methallyl, Cinnamyl oder insbesondere Allyl.

Ein wasserlöslicher niederaliphatischer oder 5- oder 6-gliedriger cycloaliphatischer Aether ist beispielsweise 1,2-Dimethoxyäthan, Dioxan oder insbesondere Tetrahydrofuran.

Geeignete Allylgruppenakzeptoren sind beispielsweise cyclische β-Dicarbonyl-Verbindungen mit einem $pK_\alpha$-Wert von 5 oder kleiner als 5, z.B. Dimedon, Meldrum's Säure (2,2-Dimethyl-1,3-dioxan-4,5-dion) sowie Barbitursäure und ihre N-mono- und N,N'-di-substituierten Derivate, wie Barbitursäure und N,N'-Dimethylbarbitursäure. Bevorzugt als Allylgruppenakzeptoren sind Dimedon und N,N'-Dimethylbarbitursäure.

Ein geeigneter Palladium(O)-Katalysator ist beispielsweise Bis-(dibenzylidenacetonato)-palladium oder Tetrakis-(triphenylphosphin)-palladium.

Die Umsetzung wird in einem der genannten wasserlöslichen Aether, der ca. 2-15 Volumen-%, insbesondere ca. 3-8 Volumen-%, Wasser enthält, bei Raumtemperatur oder geringfügig erniedrigter oder erhöhter Temperatur, z.B. bei etwa 0° bis etwa 30°C, bevorzugt bei

etwa 0 bis 20°C, durchgeführt. Die Reaktion wird mit 1,5-3 Moläquivalenten, insbesondere 1,8-2,4 Moläquivalenten, des Allylgruppenakzeptors in Gegenwart von ca. 1-5 Mol-%, insbesondere 1-3 Mol-%, Palladium(0)-Katalysator und vorzugsweise in Gegenwart von 10-50 Mol-%, insbesondere 15-30 Mol-%, Triphenylphosphin, erforderlichenfalls in einer Inertgasatmosphäre, z.B. in einer Stickstoff-oder Argonatmosphäre, durchgeführt.

Ueberraschenderweise wurde festgestellt, dass das Reaktionsprodukt, (5R,6S)-2-Aminomethyl-6-[(1R)-1-hydroxyäthyl]-2-penem-3-carbonsäure, unabhängig von der Art des verwendeten wasserlöslichen Aethers, als kristallines Monohydrat-Aethersolvat ausfällt, bei Verwendung von Tetrahydrofuran als Aetherkomponente des Lösungsmittels beispielsweise als gut filtrierbares kristallines Monohydrat-Tetrahydrofuran-Hemisolvat. Die Solvate fallen in nahezu quantitativer Ausbeute und analysenrein an. Wegen der hohen Reinheit der Monohydrat-Aethersolvate, insbesondere des Monohydrat-Tetrahydrofuran-Hemisolvats, sind weitere Reinigungsschritte unnötig, und diese Zwischenprodukte können direkt in die erfindungsgemässen Verfahren zur Herstellung des (5R,6S)-2-Aminomethyl-6-[(1R)-1-hydroxyäthyl]-2-penem-3-carbonsäure-Methansulfonats und des (5R,6S)-2-Aminomethyl-6-[(1R)-1-hydroxyäthyl]-2-penem-3-carbonsäure-Monohydrats eingesetzt werden. So ist bei Verwendung des Monohydrat-Tetrahydrofuran-Hemisolvats als Ausgangsverbindung das (5R,6S)-2-Aminomethyl-6-[(1R)-1-hydroxyäthyl]-2-penem-3-carbonsäure-Monohydrat in sehr guten Ausbeuten und in hoher Reinheit (zur Herstellung einer pharmazeutischen Form sind nur wenige unproblematische Reinigungsschritte, wie Behandlung mit Aktivkohle und Sterilfiltration, erforderlich) erhältlich.

Die Erfindung betrifft daher ferner insbesondere das (5R,6S)-2-Aminomethyl-6-[(1R)-1-hydroxyäthyl]-2-penem-3-carbonsäure-Monohydrat-Tetrahydrofuran-Hemisolvat in amorpher und kristalliner Form. Die kristalline Form des Solvats ist durch die nachstehenden

Gitterabstände (d-Werte) über 2,9 Ångström und relativen Linienintensitäten ihres Röntgenpulverdiagrammes (Kamera nach Guinier, Strahlungsquelle Kupfer-$K_{\alpha 1}$) charakterisiert:

| d-Werte (Ångström) | relative Intensität |
|---|---|
| 10.2 | sehr stark |
| 9.6 | sehr stark |
| 6.1 | mittel |
| 5.76 | sehr stark |
| 5.11 | mittel |
| 4.82 | stark |
| 4.78 | stark |
| 4.64 | stark |
| 4.41 | sehr schwach |
| 4.07 | mittel |
| 3.98 | stark |
| 3.82 | sehr stark |
| 3.72 | stark |
| 3.58 | sehr stark |
| 3.29 | sehr stark |
| 3.13 | mittel |
| 3.07 | sehr schwach |
| 3.03 | schwach |
| 2.98 | mittel |
| 2.96 | sehr schwach |
| 2.94 | schwach |

Die Ausgangsverbindungen der Formel I sind bekannt, beispielsweise aus der Deutschen Offenlegungsschrift Nr. 3431980, oder können in Analogie zu den dort beschriebenen Verfahren hergestellt werden.

Die Erfindung betrifft auch das Verfahren zur Herstellung des kristallinen Monohydrats der (5R,6S)-2-Aminomethyl-6-[(1R)-1-hydroxyäthyl]-2-penem-3-carbonsäure, dadurch gekennzeichnet, dass man eine Verbindung der Formel I in ein (5R,6S)-2-Aminomethyl-6-[(1R)-1-hydroxyäthyl]-2-penem-3-carbonsäure-Monohydrat-Aethersolvat

überführt, eine Lösung desselben wie angegeben, z.B. über ein Sulfonat-Zwischenprodukt, in eine übersättigte Lösung der freien Penem-Verbindung überführt und das Produkt zur Kristallisation bringt.

Die Erfindung betrifft weiterhin pharmazeutische Präparate, welche eine therapeutisch wirksame Menge des kristallinen (5R,6S)-2-Aminomethyl-6-[(1R)-1-hydroxyäthyl]-2-penem-3-carbonsäure-Monohydrats zusammen oder im Gemisch mit anorganischen oder organischen, festen oder flüssigen, pharmazeutisch annehmbaren Trägerstoffen enthalten, die sich zur parenteralen, d.h. z.B. intramuskulären, intravenösen, subcutanen oder intraperitonealen, Verabreichung eignen.

Zur parenteralen Verabreichung eignen sich in erster Linie Infusionslösungen, vorzugsweise isotonische wässrige Lösungen oder Suspensionen, wobei diese z.B. aus Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs- und Lösungsverfahren, hergestellt. Das erfindungsgemäss erhältliche kristalline (5R,6S)-2-Aminomethyl-6-[(1R)-1-hydroxyäthyl]-2-penem-3-carbonsäure-Monohydrat ist gut rieselfähig und kann als solches oder nach Zumischung zusätzlicher Trägerstoffe, wie Mannit, maschinell unter aseptischen Bedingungen direkt in der gewünschten Menge in Vials oder Ampullen abgefüllt werden. Die Präparate der vorliegenden Erfindung enthalten von etwa 0,1 % bis 100 %, Zubereitungen in Ampullen von etwa 50 % bis zu 100 % des Aktivstoffs.

Je nach Art der Infektion und individuellem Zustand des infizierten Organismus verwendet man tägliche parenterale Dosen von etwa 100 mg bis etwa 5 g des Wirkstoffs zur Behandlung von Warmblütern (Menschen und Tiere) von etwa 70 kg Gewicht.

Die Verwendung der erfindungsgemässen kristallinen Penem-Verbindung zur therapeutischen Behandlung des menschlichen und tierischen Körpers, insbesondere als antibakterielles Antibiotikum, bildet ebenfalls einen Gegenstand der vorliegenden Erfindung.

Die folgenden Beispiele dienen zur Illustration der Erfindung. Temperaturen werden in Celsiusgraden angegeben.

Experimenteller Teil

Beschreibung der Röntgenpulverdiagramme:

Zur Bestimmung der Netzebenenabstände (d-Werte) wurde das Beugungsbild auf Film registriert. Die Aufnahme erfolgte in Transmission mit einer Guinier-Kamera (Enraf-Nonius FR 552) und Kupfer-K-alpha-1-Strahlung (Wellenlänge = 1,54050 Å). Als Eichsubstanz wurde Quarz verwendet, dessen d-Werte aus a = 4,913 Å und c = 5,405 Å (PDF 5-490) berechnet wurden. Die beiliegenden Tabellen enthalten die d-Werte der stärksten Linien, zusammen mit den von Auge geschätzten relativen Linienintensitäten.

Beispiel 1: (5R,6S)-2-Aminomethyl-6-[(1R)-1-hydroxyäthyl]-2-penem-3-carbonsäure-Monohydrat-Tetrahydrofuran-Hemisolvat

45,2 g (100 mMol) (5R,6S)-2-Allyloxycarbonylaminomethyl-6-[(1R)-1-allyloxycarbonyloxyäthyl]-2-penem-3-carbonsäureallylester, 28,0 g (180 mMol) N,N'-Dimethylbarbitursäure und 5,2 g (20 mMol) Triphenylphosphin werden in einem Gemisch von 500 ml Tetrahydrofuran und 25 ml Wasser gelöst. Die Lösung wird während 15 Minuten mit Argon gespült, dann bei 0 - 5° mit 1,6 g (1,4 mMol) Tetrakis-(triphenylphosphin)-palladium versetzt und während 2,5 Stunden ausgerührt,

wobei die Temperatur allmählich auf Raumtemperatur angehoben wird. Der kristalline Niederschlag wird abfiltriert, mit Tetrahydrofuran gewaschen und am Hochvakuum getrocknet. DC ($H_2O$, OPTI-UP $C_{12}$), $R_f$ = 0,48. Smp. 127°C (Zers.).

$C_9H_{12}N_2O_4S \cdot H_2O \cdot 0{,}5\ C_4H_8O$ (Molekulargewicht 298,3)

| | berechnet (%) | gefunden (%) |
|---|---|---|
| C | 44,3 | 44,1 |
| H | 6,1 | 6,1 |
| N | 9,4 | 9,4 |
| O | 29,5 | 29,6 |
| S | 10,7 | 10,3 |
| THF | 12,1 | 12,3 |
| $H_2O$ | 6,8 | 6,7 |

Den 21 stärksten Linien im Röntgenpulverdiagramm mit d-Werten über 2,9 Ångström entsprechen folgende Netzebenenabstände und relative Intensitäten:

| d-Werte (Ångström) | relative Intensität |
|---|---|
| 10.2 | sehr stark |
| 9.6 | sehr stark |
| 6.1 | mittel |
| 5.76 | sehr stark |
| 5.11 | mittel |
| 4.82 | stark |
| 4.78 | stark |
| 4.64 | stark |
| 4.41 | sehr schwach |
| 4.07 | mittel |
| 3.98 | stark |
| 3.82 | sehr stark |
| 3.72 | stark |
| 3.58 | sehr stark |

| d-Werte (Ångström) | relative Intensität |
|---|---|
| 3.29 | sehr stark |
| 3.13 | mittel |
| 3.07 | sehr schwach |
| 3.03 | schwach |
| 2.98 | mittel |
| 2.96 | sehr schwach |
| 2.94 | schwach |

Beispiel 2: (5R,6S)-2-Aminomethyl-6-[(1R)-1-hydroxyäthyl]-2-penem-3-carbonsäure-methansulfonat

22,3 g (75 mMol) des (5R,6S)-2-Aminomethyl-6-[(1R)-1-hydroxyäthyl]-2-penem-3-carbonsäure-Monohydrat-Tetrahydrofuran-Hemisolvats werden in einem Gemisch von 20 ml Wasser und 60 ml absol. Aethanol bei 5-10° suspendiert und durch langsame Zugabe einer Lösung von 5 ml (77 mMol) Methansulfonsäure in 10 ml Wasser gelöst. Die leicht trübe Lösung wird filtriert. Das Filtrat wird mit 150 ml absol. Aethanol versetzt und bei 0-5° gerührt, wobei die Kristallisation einsetzt, welche schliesslich durch mehrstündiges Rühren bei -25° vervollständigt wird. Das Produkt wird abfiltriert, mit kaltem absol. Aethanol gewaschen und am Hochvakuum getrocknet: flaumiges, weisses Kristallisat, Smp. 129-131° (unter Zersetzung).

$C_9H_{12}N_2O_4S \cdot CH_4O_3S$          (Molekulargewicht: 340,4)

| | berechnet (%) | gefunden (%) |
|---|---|---|
| C | 35,29 | 35,3 |
| H | 4,74 | 4,8 |
| N | 8,23 | 8,4 |
| O | 32,91 | 33,2 |
| S | 18,84 | 18,3 |

Den 32 stärksten Linien im Röntgenpulverdiagramm mit d-Werten über 3,0 Ångström entsprechen folgende Netzebenenabstände und relative Intensitäten:

| d-Werte (Ångström) | relative Intensität |
|---|---|
| 15.0 | sehr stark |
| 13.1 | schwach |
| 10.7 | mittel |
| 9.2 | sehr stark |
| 8.8 | sehr schwach |
| 7.5 | schwach |
| 6.7 | schwach |
| 6.5 | mittel |
| 5.86 | sehr schwach |
| 5.32 | schwach |
| 4.97 | schwach |
| 4.86 | mittel |
| 4.77 | mittel |
| 4.61 | stark |
| 4.57 | schwach |
| 4.41 | mittel |
| 4.18 | schwach |
| 4.09 | schwach |
| 4.01 | sehr schwach |
| 3.94 | stark |
| 3.69 | stark |
| 3.61 | mittel |
| 3.58 | mittel |
| 3.52 | schwach |
| 3.43 | mittel |
| 3.40 | schwach |
| 3.31 | stark |
| 3.24 | mittel |
| 3.18 | sehr schwach |
| 3.12 | sehr schwach |
| 3.06 | mittel |
| 3.02 | mittel |

Beispiel 3: (5R,6S)-2-Aminomethyl-6-[(1R)-1-hydroxyäthyl]-2-penem-3-carbonsäure-Monohydrat (Herstellung über das Sulfonat)

a. Aus dem Monohydrat-Tetrahydrofuran-Hemisolvat (in wässrigem Aethanol)

Eine Suspension von 29,8 g (100 mMol) des (5R,6S)-2-Aminomethyl-6-[(1R)-1-hydroxyäthyl]-2-penem-3-carbonsäure-Monohydrat-Tetrahydrofuran-Hemisolvats in 60 ml Wasser wird bei 10° durch langsame Zugabe von 32 ml wässriger (3M) Methansulfonsäure (96 mMol) gelöst. Die Lösung wird während 15 Minuten mit 3 g Aktivkohle verrührt und filtriert. Das Filtrat (inkl. 20 ml Spüllösung) wird bei 5-10° mit 120 ml absolutem Aethanol verdünnt, dann mit einer Lösung von 3,47 ml (25 mMol) Triäthylamin in 60 ml absol. Aethanol versetzt, gegebenenfalls angeimpft und 30 Minuten gerührt, wobei eine Suspension entsteht. Danach wird bei gleicher Temperatur innert 90 Minuten eine Lösung von 9,3 ml (66 mMol) Triäthylamin in 158 ml Aethanol dazugetropft. Die Suspension wird bis -25° abgekühlt, 2 h gerührt und schliesslich filtriert, mit kaltem Aethanol (96 %) gewaschen und am Hochvakuum bei 30° getrocknet. Die Titelverbindung fällt farblos und grobkristallin an. Smp. 133° (Zers.). DC ($H_2O$, OPTI-UP $C_{12}$), $R_f$ = 0,48.

$C_9H_{12}N_2O_4S \cdot H_2O$ (Molekulargewicht: 262,3)

| | berechnet (%) | gefunden (%) |
|---|---|---|
| C | 41,3 | 41,2 |
| H | 5,4 | 5,4 |
| N | 10,7 | 10,7 |
| O | 30,5 | 30,7 |
| S | 12,2 | 12,0 |
| $H_2O$ | 6,9 | 6,7 |

Den 23 stärksten Linien im Röntgenpulverdiagramm mit d-Werten über 2,8 Ångström entsprechen folgende Netzebenenabstände und relative Intensitäten:

| d-Werte (Ångström) | relative Intensität |
|---|---|
| 8.3 | mittel |
| 6.6 | sehr stark |
| 5.46 | stark |
| 4.97 | stark |
| 4.75 | sehr stark |
| 4.54 | stark |
| 4.19 | mittel |
| 4.12 | schwach |
| 3.85 | sehr stark |
| 3.79 | stark |
| 3.67 | schwach |
| 3.58 | stark |
| 3.40 | stark |
| 3.33 | sehr schwach |
| 3.28 | schwach |
| 3.27 | mittel |
| 3.24 | mittel |
| 3.17 | sehr stark |
| 3.13 | sehr schwach |
| 2.99 | sehr stark |
| 2.98 | mittel |
| 2.93 | sehr stark |
| 2.88 | schwach |

b. Aus dem Monohydrat-Tetrahydrofuran-Hemisolvat (in wässrigem Isopropanol)

Eine Suspension von 29,8 g (100 mMol) des (5R,6S)-2-Aminomethyl-6-[(1R)-1-hydroxyäthyl]-2-penem-3-carbonsäure-Monohydrat-Tetrahydrofuran-Hemisolvats in 60 ml Wasser wird bei 10° durch langsame Zugabe von 32 ml wässriger (3M) Methansulfonsäure (96 mMol) gelöst. Die Lösung wird während 15 Minuten mit 3 g Aktivkohle verrührt und filtriert. Das Filtrat (inkl. 20 ml Spüllösung) wird bei 5-10° mit 120 ml Isopropanol verdünnt, dann mit einer Lösung von 1,5 ml (25 mMol) Aethanolamin in 60 ml Isopropanol versetzt, gegebenenfalls

0243921

angeimpft und 30 Minuten gerührt, wobei eine Suspension entsteht. Danach wird bei gleicher Temperatur innert 90 Minuten eine Lösung von 4,0 ml Aethanolamin (66 mMol) in 160 ml Isopropanol zugetropft. Die Suspension wird bis -25° abgekühlt, 2 h gerührt und filtriert. Das Monohydrat wird mit wenig kaltem Isopropanol, der 5 % Wasser enthält, gewaschen und unter Vakuum bei 30° getrocknet. Das Produkt fällt in farbloser grobkristalliner Form an. Smp. 129° (Zers.). DC ($H_2O$, OPTI-UP $C_{12}$), $R_f$ = 0.48.

$C_9H_{12}N_2O_4S \cdot H_2O$ (Molekulargewicht: 262.3)

| | berechnet (%) | gefunden (%) |
|---|---|---|
| C | 41.3 | 41.2 |
| H | 5.4 | 5.5 |
| N | 10.7 | 10.4 |
| O | 30.5 | 30.6 |
| S | 12.2 | 12.1 |
| $H_2O$ | 6.9 | 7.1 |

Das Röntgenpulverdiagramm des Produktes ist mit dem in Beispiel 3a beschriebenen Röntgenpulverdiagramm identisch.

c. Aus dem Methansulfonat

17,1 g (50 mMol) (5R,6S)-2-Aminomethyl-6-[(1R-1-hydroxyäthyl]-2-penem-3-carbonsäure-methansulfonat werden in einem Gemisch von 40 ml Wasser und 20 ml absol. Aethanol bei 10° gelöst. Zur Lösung werden unter Rühren sofort 30 ml 0,4 M Triäthylamin-Lösung in absol. Aethanol bei 5-10° zugegeben. Nach Animpfen wird bei 5-10° während 30 Minuten weitergerührt, wobei sich eine Kristallsuspension bildet. Nun werden innert 90 Minuten weitere ca. 95 ml 0,4 M Triäthylamin-Lösung in absol. Aethanol zugetropft, bis pH 5 erreicht ist. Die Suspension wird bis -25° abgekühlt und während 2-4 Stunden bei dieser Temperatur gerührt. Das kristalline Produkt wird filtriert, mit kaltem Aethanol (96 %) gewaschen und am Hochvakuum getrocknet. DC: (Wasser, OPTI-UP $C_{12}$), $R_f$ = 0,48. Der Schmelzpunkt, die Kristallform und das Röntgenpulverdiagramm des Produktes sind mit den entsprechenden Daten des in Beispiel 3a) beschriebenen Produktes identisch.

Beispiel 4: (5R,6S)-2-Aminomethyl-6-[(1R)-1-hydroxyäthyl]-2-penem-3-carbonsäure-Monohydrat (direkte Herstellung aus dem Monohydrat-THF-Hemisolvat)

29.8 g (100 mMol) (5R,6S)-2-Aminomethyl-6-[(1R)-1-hydroxyäthyl]-2-penem-3-carbonsäure-Monohydrat-Tetrahydrofuran-Hemisolvat werden bei 45-50° in 250 ml Wasser gelöst. Die warme Lösung wird während 10 min. mit 3 g Aktivkohle verrührt und anschliessend filtriert. Die vereinigten Filtrate (inkl. 25 ml Spülwasser) werden bei 45-50° rasch mit 1200 ml warmem absolutem Aethanol versetzt. Die anfänglich noch klare Lösung wird angeimpft und innert 2 h gleichmässig bis -25° abgekühlt. Die Kristallsuspension wird weitere 2 h bei -25° gerührt und schliesslich filtriert. Das Produkt wird mit wenig kaltem Aethanol (96 %) gewaschen und bei 30° unter Vakuum getrocknet. Man erhält ein flaumiges, farbloses Kristallisat. Smp. 132° (unter Zersetzung). DC ($H_2O$, OPTI-UP $C_{12}$), $R_f$ = 0.48.

$C_9H_{12}N_2O_4S \cdot H_2O$ (Molekulargewicht: 262.3)

| | berechnet (%) | gefunden (%) |
|---|---|---|
| C | 41.3 | 41.1 |
| H | 5.4 | 5.5 |
| N | 10.7 | 10.6 |
| O | 30.5 | 30.6 |
| S | 12.2 | 12.1 |
| $H_2O$ | 6.9 | 7.0 |

Das Röntgenpulverdiagramm des Produktes ist mit dem in Beispiel 3a beschriebenen Röntgenpulverdiagramm identisch.

Beispiel 5: Bestimmung der thermischen Stabilität und der Wasseraufnahme aus Luft

Zur Bestimmung der thermischen Stabilität und der Wasseraufnahme aus Luft werden Proben des kristallinen (5R,6S)-2-Aminomethyl-6-[(1R)-1-hydroxyäthyl]-2-penem-3-carbonsäure-Monohydrats (Verbindung 1) und der aus der Deutschen Offenlegungsschrift Nr. 3431980 vorbekannten amorphen (5R,6S)-2-Aminomethyl-6-[(1R)-1-hydroxyäthyl]-2-penem-3-carbonsäure (Verbindung 2) verwendet.

Zur Bestimmung der thermischen Lagerstabilität werden die Testverbindungen in geschlossenen Glasröhrchen in einem thermostatischen Oelbad auf 50° erwärmt und 14 Tage bei dieser Temperatur gehalten. Nach 1, 3, 7 und 14 Tagen werden Proben entnommen, die mittels Flüssigkeitschromatographie (LC) auf den Gehalt an unzersetztem Penem geprüft werden. Von einzelnen Proben wird auch die Extinktion bei 425 nm (c = 5,0; Wasser) gemessen.

Weiterhin wird die Lagerstabilität der Testverbindungen bei 35° und 95 % relativer Luftfeuchtigkeit während 7 Tagen bestimmt. Zur Untersuchung werden wiederum Gehaltsbestimmungen mittels Flüssigkeitschromatographie (LC) und Extinktionsmessungen bei 425 nm herangezogen. Ausserdem wird die Wasseraufnahme der Testverbindungen während 7 Tagen bestimmt. Die Wasserbestimmungen erfolgen nach der bekannten Methode von Karl Fischer.

Die Ergebnisse sind in den folgenden Tabellen zusammengefasst:

a. Stabilität bei 50° (geschlossene Glasröhrchen)

| Tag | LC-Gehalt Verbindung 1 | LC-Gehalt Verbindung 2 | Extinktion Verbindung 1 | Extinktion Verbindung 2 |
|---|---|---|---|---|
| 0 | 94,0 % | 94,7 % | 0,04 | 0,29 |
| 1 | 93,6 | 90,7 | 0,09 | 0,51 |
| 3 | 94,0 | 86,7 | - | - |
| 7 | 94,1 | 82,1 | 0,09 | 1,41 |
| 14 | 93,8 | 77,4 | - | - |

b. Stabilität bei 35° und 95 % relativer Luftfeuchtigkeit

| Tag | LC-Gehalt Verbindung 1 | LC-Gehalt Verbindung 2 | Extinktion Verbindung 1 | Extinktion Verbindung 2 | Wasser-Geghalt Verbindung 1 | Wasser-Geghalt Verbindung 2 |
|---|---|---|---|---|---|---|
| 0 | 94,0 % | 94,7 % | 0,04 | 0,29 | 7,1 % | 2,7 % |
| 7 | 94,2 | 17,0 | 0,10 | >3,50 | 7,6 | 15.7 |

Die Ergebnisse belegen, dass das erfindungsgemässe kristalline Hydrat eine erheblich höhere Stabilität bei Wärme- und Feuchtigkeitseinwirkung aufweist als das vorbekannte amorphe Produkt.

Beispiel 6: Trockenampullen oder Vials, enthaltend kristallines (5R,6S)-2-Aminomethyl-6-[(1R)-1-hydroxyäthyl]-2-penem-3-carbonsäure-Monohydrat als Wirksubstanz, werden wie folgt hergestellt:

Zusammensetzung (für 1 Ampulle oder Vial):

| | |
|---|---|
| Wirksubstanz | 0,5 g |
| Mannit | 0,05 g |

Die Wirksubstanz und das Mannit werden unter aseptischen Bedingungen abgewogen, in 10 ml-Ampullen oder 10 ml-Vials abgefüllt und die Ampullen bzw. Vials verschlossen und geprüft.

Patentansprüche für alle Vertragsstaaten ausser AT, ES, GR

1. Kristallines (5R,6S)-2-Aminomethyl-6-[(1R)-1-hydroxyäthyl]-2-penem-3-carbonsäure-Monohydrat.

2. Kristallines (5R,6S)-2-Aminomethyl-6-[(1R)-1-hydroxyäthyl]-2-penem-3-carbonsäure-Monohydrat gemäss Anspruch 1, gekennzeichnet durch die nachfolgenden Gitterabstände (d-Werte über 2,8 Ångström) und relativen Linienintensitäten seines Röntgenpulverdiagramms (Kamera nach Guinier, Strahlungsquelle Kupfer-$K_{\alpha 1}$):

| d-Werte (Ångström) | relative Intensität |
|---|---|
| 8.3 | mittel |
| 6.6 | sehr stark |
| 5.46 | stark |
| 4.97 | stark |
| 4.75 | sehr stark |
| 4.54 | stark |
| 4.19 | mittel |
| 4.12 | schwach |
| 3.85 | sehr stark |
| 3.79 | stark |
| 3.67 | schwach |
| 3.58 | stark |
| 3.40 | stark |
| 3.33 | sehr schwach |
| 3.28 | schwach |
| 3.27 | mittel |
| 3.24 | mittel |
| 3.17 | sehr stark |
| 3.13 | sehr schwach |
| 2.99 | sehr stark |
| 2.98 | mittel |
| 2.93 | sehr stark |
| 2.88 | schwach |

3. Kristallines (5R,6S)-2-Aminomethyl-6-[(1R)-1-hydroxyäthyl]-2-penem-3-carbonsäure-Monohydrat gemäss Anspruch 1, dessen Herstellung dadurch gekennzeichnet ist, dass man eine übersättigte Lösung eines (5R,6S)-2-Aminomethyl-6-[(1R)-1-hydroxyäthyl]-2-penem-3-carbonsäure-Monohydrat-Aethersolvats in einem wasserhaltigen $C_1$-$C_4$-Alkanol herstellt, oder ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, die Lösung des gebildeten Sulfonat-Salzes, gegebenenfalls nach vorhergehender Isolierung des Sulfonats aus dem Reaktionsgemisch und anschliessender Wiederauflösung des Isolats in Wasser oder in einem Gemisch bestehend aus Wasser und einem $C_1$-$C_4$-Alkanol, durch Zugabe äquimolarer Mengen einer organischen Stickstoffbase und eines $C_1$-$C_4$-Alkanols in eine übersättigte Lösung der freien Penem-Verbindung überführt, das Produkt zur Kristallisation bringt, und die Kristalle isoliert und trocknet.

4. Verfahren zur Herstellung des (5R,6S)-2-Aminomethyl-6-[(1R)-1-hydroxyäthyl]-2-penem-3-carbonsäure-Monohydrats, dadurch gekennzeichnet, dass man eine übersättigte Lösung eines (5R,6S)-2-Aminomethyl-6-[(1R)-1-hydroxyäthyl]-2-penem-3-carbonsäure-Monohydrat-Aethersolvats in einem wasserhaltigen $C_1$-$C_4$-Alkanol herstellt, oder ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, die Lösung des gebildeten Sulfonat-Salzes, gegebenenfalls nach vorhergehender Isolierung des Sulfonats aus dem Reaktionsgemisch und anschliessender Wiederauflösung des Isolats in Wasser oder in einem Gemisch bestehend aus Wasser und einem $C_1$-$C_4$-Alkanol, durch Zugabe äquimolarer Mengen einer organischen Stickstoffbase und eines $C_1$-$C_4$-Alkanols in eine übersättigte Lösung der freien Penem-Verbindung überführt, das Produkt zur Kristallisation bringt, und die Kristalle isoliert und trocknet.

5. Pharmazeutisches Präparat enthaltend (5R,6S)-2-Aminomethyl-6-[(1R)-1-hydroxyäthyl]-2-penem-3-carbonsäure-Monohydrat in kristalliner Form.

6. Kristallines (5R,6S)-2-Aminomethyl-6-[(1R)-1-hydroxyäthyl]-2-penem-3-carbonsäure-Monohydrat zur Anwendung in einem Verfahren zur therapeutischen Behandlung des menschlichen und tierischen Körpers.

7. Verwendung von kristallinem (5R,6S)-2-Aminomethyl-6-[(1R)-1-hydroxyäthyl]-2-penem-3-carbonsäure-Monohydrat zur Herstellung von pharmazeutischen Präparaten.

8. Sulfonate der (5R,6S)-2-Aminomethyl-6-[(1R)-1-hydroxyäthyl]-2-penem-3-carbonsäure in amorpher und in kristalliner Form.

9. Sulfonate gemäss Anspruch 8, worin das Sulfonat ein gegebenenfalls durch Halogen substituiertes $C_1$-$C_4$-Alkansulfonat oder ein gegebenenfalls durch $C_1$-$C_4$-Alkyl oder Halogen substituiertes Benzolsulfonat ist.

10. Das (5R,6S)-2-Aminomethyl-6-[(1R)-1-hydroxyäthyl]-2-penem-3-carbonsäure-methansulfonat gemäss Anspruch 8.

11. Kristallines (5R,6S)-2-Aminomethyl-6-[(1R)-1-hydroxyäthyl]-2-penem-3-carbonsäure-methansulfonat gemäss Anspruch 10, gekennzeichnet durch die nachfolgenden Gitterabstände (d-Werte über 3,0 Ångström) und relativen Linienintensitäten seines Röntgenpulverdiagramms (Kamera nach Guinier, Strahlungsquelle: Kupfer-$K_{\alpha 1}$):

| d-Werte (Ångström) | relative Intensität |
|---|---|
| 15.0 | sehr stark |
| 13.1 | schwach |
| 10.7 | mittel |
| 9.2 | sehr stark |
| 8.8 | sehr schwach |

| d-Werte (Ångström) | relative Intensität |
|---|---|
| 7.5 | schwach |
| 6.7 | schwach |
| 6.5 | mittel |
| 5.86 | sehr schwach |
| 5.32 | schwach |
| 4.97 | schwach |
| 4.86 | mittel |
| 4.77 | mittel |
| 4.61 | stark |
| 4.57 | schwach |
| 4.41 | mittel |
| 4.18 | schwach |
| 4.09 | schwach |
| 4.01 | sehr schwach |
| 3.94 | stark |
| 3.69 | stark |
| 3.61 | mittel |
| 3.58 | mittel |
| 3.52 | schwach |
| 3.43 | mittel |
| 3.40 | schwach |
| 3.31 | stark |
| 3.24 | mittel |
| 3.18 | sehr schwach |
| 3.12 | sehr schwach |
| 3.06 | mittel |
| 3.02 | mittel |

12. Verfahren zur Herstellung von kristallinen Sulfonaten der (5R,6S)-2-Aminomethyl-6-[(1R)-1-hydroxyäthyl]-2-penem-3-carbonsäure, dadurch gekennzeichnet, dass man ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, durch Zugabe

eines Ueberschusses des $C_1$-$C_4$-Alkanols eine übersättigte Lösung herstellt, das Produkt zur Kristallisation bringt, und die Kristalle isoliert und trocknet.

13. Monohydrat-Aethersolvate der (5R,6S)-2-Aminomethyl-6-[(1R)-1-hydroxyäthyl]-2-penem-3-carbonsäure in amorpher und kristalliner Form.

14. Monohydrat-Aethersolvate gemäss Anspruch 13, dadurch gekennzeichnet, dass der Aether ein wasserlöslicher niederaliphatischer oder 5- oder 6-gliedriger cycloaliphatischer Aether ist.

15. Monohydrat-Aethersolvate gemäss Anspruch 14, dadurch gekennzeichnet, dass der Aether 1,2-Dimethoxyäthan, Dioxan oder Tetrahydrofuran ist.

16. Kristallines (5R,6S)-2-Aminomethyl-6-[(1R)-1-hydroxyäthyl]-2-penem-3-carbonsäure-Monohydrat-Tetrahydrofuran-Hemisolvat gemäss Anspruch 15, gekennzeichnet durch die nachfolgenden Gitterabstände (d-Werte über 2,9 Ångström) und relativen Linienintensitäten seines Röntgenpulverdiagramms (Kamera nach Guinier, Strahlungsquelle: Kupfer-$K_{\alpha 1}$):

| d-Werte (Ångström) | relative Intensität |
|---|---|
| 10.2 | sehr stark |
| 9.6 | sehr stark |
| 6.1 | mittel |
| 5.76 | sehr stark |
| 5.11 | mittel |
| 4.82 | stark |
| 4.78 | stark |
| 4.64 | stark |
| 4.41 | sehr schwach |
| 4.07 | mittel |
| 3.98 | stark |
| 3.82 | sehr stark |

| d-Werte (Ångström) | relative Intensität |
|---|---|
| 3.72 | stark |
| 3.58 | sehr stark |
| 3.29 | sehr stark |
| 3.13 | mittel |
| 3.07 | sehr schwach |
| 3.03 | schwach |
| 2.98 | mittel |
| 2.96 | sehr schwach |
| 2.94 | schwach |

17. Verfahren zur Herstellung von (5R,6S)-2-Aminomethyl-6-[(1R)-1-hydroxyäthyl]-2-penem-3-carbonsäure-Monohydrat-Aethersolvaten, dadurch gekennzeichnet, dass man eine Verbindung der Formel

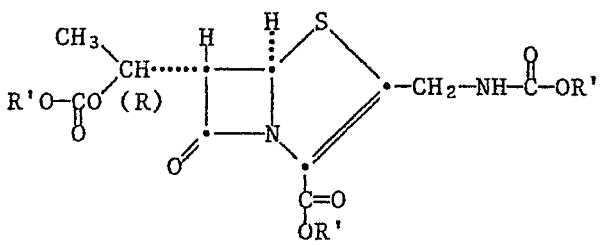

(I)

worin R' für gegebenenfalls substituiertes Allyl steht, in einem Gemisch bestehend aus einem wasserlöslichen niederaliphatischen oder 5- oder 6-gliedrigen cycloaliphatischen Aether mit einem Allylgruppenakzeptor in Gegenwart eines geeigneten Palladium(0)-Katalysators und gegebenenfalls in Gegenwart von Triphenylphosphin umsetzt.

Patentansprüche für die Vertragsstaaten AT, ES, GR

1. Verfahren zur Herstellung des kristallinen (5R,6S)-2-Aminomethyl-6-[(1R)-1-hydroxyäthyl]-2-penem-3-carbonsäure-Monohydrats, dadurch gekennzeichnet, dass man eine übersättigte Lösung eines (5R,6S)-2-Aminomethyl-6-[(1R)-1-hydroxyäthyl]-2-penem-3-carbonsäure-Monohydrat-Aethersolvats in einem wasserhaltigen $C_1$-$C_4$-Alkanol herstellt, oder ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, die Lösung des gebildeten Sulfonat-Salzes, gegebenenfalls nach vorhergehender Isolierung des Sulfonats aus dem Reaktionsgemisch und anschliessender Wiederauflösung des Isolats in Wasser oder in einem Gemisch bestehend aus Wasser und einem $C_1$-$C_4$-Alkanol, durch Zugabe äquimolarer Mengen einer organischen Stickstoffbase und eines $C_1$-$C_4$-Alkanols in eine übersättigte Lösung der freien Penem-Verbindung überführt, das Produkt zur Kristallisation bringt, und die Kristalle isoliert und trocknet.

2. Verfahren gemäss Anspruch 1, zur Herstellung des kristallinen (5R,6S)-2-Aminomethyl-6-[(1R)-1-hydroxyäthyl]-2-penem-3-carbonsäure-Monohydrats, gekennzeichnet durch die nachfolgenden Gitterabstände (d-Werte über 2,8 Ångström) und relativen Linienintensitäten seines Röntgenpulverdiagramms (Kamera nach Guinier, Strahlungsquelle Kupfer-$K_{\alpha 1}$):

| d-Werte (Ångström) | relative Intensität |
|---|---|
| 8.3 | mittel |
| 6.6 | sehr stark |
| 5.46 | stark |
| 4.97 | stark |
| 4.75 | sehr stark |
| 4.54 | stark |
| 4.19 | mittel |

| d-Werte (Ångström) | relative Intensität |
|---|---|
| 4.12 | schwach |
| 3.85 | sehr stark |
| 3.79 | stark |
| 3.67 | schwach |
| 3.58 | stark |
| 3.40 | stark |
| 3.33 | sehr schwach |
| 3.28 | schwach |
| 3.27 | mittel |
| 3.24 | mittel |
| 3.17 | sehr stark |
| 3.13 | sehr schwach |
| 2.99 | sehr stark |
| 2.98 | mittel |
| 2.93 | sehr stark |
| 2.88 | schwach |

3. Verfahren gemäss Anspruch 1, dadurch gekennzeichnet, dass man von einem (5R,6S)-2-Aminomethyl-6-[(1R)-1-hydroxyäthyl]-2-penem-3-carbonsäure-Monohydrat-Aethersolvat ausgeht, worin der Aether ein wasserlöslicher niederaliphatischer oder 5- oder 6-gliedriger cycloaliphatischer Aether ist.

4. Verfahren gemäss Anspruch 3, dadurch gekennzeichnet, dass man von einem (5R,6S)-2-Aminomethyl-6-[(1R)-1-hydroxyäthyl]-2-penem-3-carbonsäure-Monohydrat-Aethersolvat ausgeht, worin der Aether 1,2-Dimethoxyäthan, Dioxan oder Tetrahydrofuran ist.

5. Verfahren gemäss Anspruch 4, dadurch gekennzeichnet, dass man vom Monohydrat-Tetrahydrofuran-Hemisolvat der (5R,6S)-2-Aminomethyl-6-[(1R)-1-hydroxyäthyl]-2-penem-3-carbonsäure ausgeht.

6. Verfahren gemäss Anspruch 1, dadurch gekennzeichnet, dass man das (5R,6S)-2-Aminomethyl-6-[(1R)-1-hydroxyäthyl]-2-penem-3-carbonsäure-Monohydrat-Aethersolvat bei Raumtemperatur oder bei bis 50°C

erhöhter Temperatur in Wasser löst, durch Zugabe eines Ueberschusses des $C_1$-$C_4$-Alkanols eine übersättigte Lösung herstellt und die übersättigte Lösung auf 0° bis -30°C abkühlt.

7. Verfahren gemäss Anspruch 1, dadurch gekennzeichnet, dass das (5R,6S)-2-Aminomethyl-6-[(1R)-1-hydroxyäthyl]-2-penem-3-carbonsäure-Monohydrat-Aethersolvat in einem Gemisch bestehend aus Wasser und einem $C_1$-$C_4$-Alkanol, wobei der Wassergehalt 25-75 % beträgt, mit einer Sulfonsäure umgesetzt wird.

8. Verfahren gemäss Anspruch 7, dadurch gekennzeichnet, dass die Sulfonsäure eine gegebenenfalls durch Halogen substituierte $C_1$-$C_4$-Alkansulfonsäure oder eine gegebenenfalls durch $C_1$-$C_4$-Alkyl oder Halogen substituierte Benzolsulfonsäure ist.

9. Verfahren gemäss Anspruch 7, dadurch gekennzeichnet, dass man das gebildete Sulfonatsalz durch Zugabe eines Ueberschusses des $C_1$-$C_4$-Alkanols ausfällt, isoliert und in Wasser oder in einem Gemisch bestehend aus Wasser und einem $C_1$-$C_4$-Alkanol wiederauflöst.

10. Verfahren gemäss Anspruch 7, dadurch gekennzeichnet, dass man das gebildete Sulfonatsalz ohne Isolierung aus dem Reaktionsgemisch weiterverarbeitet.

11. Verfahren gemäss Anspruch 1, dadurch gekennzeichnet, dass man die Lösung des Sulfonats mit äquimolaren Mengen einer organischen Stickstoffbase versetzt und die Lösung der gebildeten freien Penem-Verbindung durch Zugabe eines Ueberschusses an $C_1$-$C_4$-Alkanol in eine übersättigte Lösung überführt.

12. Verfahren gemäss Anspruch 1, dadurch gekennzeichnet, dass das (5R,6S)-2-Aminomethyl-6-[(1R)-1-hydroxyäthyl]-2-penem-3-carbonsäure-Monohydrat-Aethersolvat hergestellt wird, indem man eine Verbindung der Formel

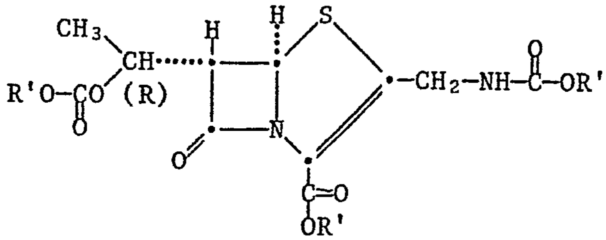

(I)

worin R' für gegebenenfalls substituiertes Allyl steht, in einem Gemisch bestehend aus einem wasserlöslichen niederaliphatischen oder 5- oder 6-gliedrigen cycloaliphatischen Aether mit einem Allylgruppenakzeptor in Gegenwart eines geeigneten Palladium(O)-Katalysators und gegebenenfalls in Gegenwart von Triphenylphosphin umsetzt.

13. Verfahren zur Herstellung von kristallinen Sulfonaten der (5R,6S)-2-Aminomethyl-6-[(1R)-1-hydroxyäthyl]-2-penem-3-carbonsäure, dadurch gekennzeichnet, dass man ein (5R,6S)-2-Aminomethyl-6-[(1R)-1-hydroxyäthyl]-2-penem-3-carbonsäure-Monohydrat-Aethersolvat in Wasser oder in einem Gemisch bestehend aus Wasser und einem $C_1$-$C_4$-Alkanol mit einer Sulfonsäure behandelt, durch Zugabe eines Ueberschusses des $C_1$-$C_4$-Alkanols eine übersättigte Lösung herstellt, das Produkt zur Kristallisation bringt, und die Kristalle isoliert und trocknet.

14. Verfahren zur Herstellung von kristallinem (5R,6S)-2-Aminomethyl-6-[(1R)-1-hydroxyäthyl]-2-penem-3-carbonsäure-methansulfonat gemäss Anspruch 13.

15. Verfahren zur Herstellung von kristallinem (5R,6S)-2-Aminomethyl-6-[(1R)-1-hydroxyäthyl]-2-penem-3-carbonsäure-methansulfonat gemäss Anspruch 14, gekennzeichnet durch die nachfolgenden Gitterabstände (d-Werte über 3,0 Ångström) und relativen Linienintensitäten seines Röntgenpulverdiagramms (Kamera nach Guinier, Strahlungsquelle: Kupfer-$K_{\alpha 1}$):

| d-Werte (Ångström) | relative Intensität |
| --- | --- |
| 15.0 | sehr stark |
| 13.1 | schwach |
| 10.7 | mittel |
| 9.2 | sehr stark |
| 8.8 | sehr schwach |
| 7.5 | schwach |
| 6.7 | schwach |
| 6.5 | mittel |
| 5.86 | sehr schwach |
| 5.32 | schwach |
| 4.97 | schwach |
| 4.86 | mittel |
| 4.77 | mittel |
| 4.61 | stark |
| 4.57 | schwach |
| 4.41 | mittel |
| 4.18 | schwach |
| 4.09 | schwach |
| 4.01 | sehr schwach |
| 3.94 | stark |
| 3.69 | stark |
| 3.61 | mittel |
| 3.58 | mittel |
| 3.52 | schwach |
| 3.43 | mittel |
| 3.40 | schwach |
| 3.31 | stark |
| 3.24 | mittel |
| 3.18 | sehr schwach |
| 3.12 | sehr schwach |
| 3.06 | mittel |
| 3.02 | mittel |

16. Verfahren zur Herstellung von (5R,6S)-2-Aminomethyl-6-[(1R)-1-hydroxyäthyl]-2-penem-3-carbonsäure-Monohydrat-Aethersolvaten, dadurch gekennzeichnet, dass man eine Verbindung der Formel

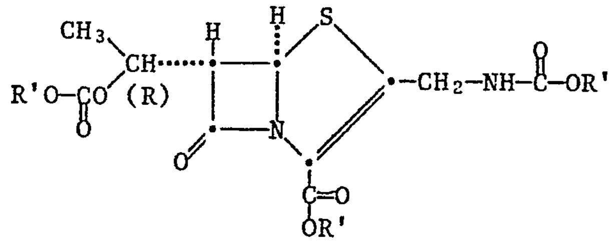

(I)

worin R' für gegebenenfalls substituiertes Allyl steht, in einem Gemisch bestehend aus einem wasserlöslichen niederaliphatischen oder 5- oder 6-gliedrigen cycloaliphatischen Aether mit einem Allylgruppenakzeptor in Gegenwart eines geeigneten Palladium(0)-Katalysators und gegebenenfalls in Gegenwart von Triphenylphosphin umsetzt.

17. Verfahren gemäss Anspruch 16, dadurch gekennzeichnet, dass man von Verbindungen der Formel I ausgeht, worin R' Allyl, Methallyl oder Cinnamyl ist.

18. Verfahren gemäss Anspruch 16, dadurch gekennzeichnet, dass der wasserlösliche niederaliphatische oder 5- oder 6-gliedrige cycloaliphatische Aether 1,2-Dimethoxyäthan, Dioxan oder Tetrahydrofuran ist.

19. Verfahren zur Herstellung von kristallinem (5R,6S)-2-Aminomethyl-6-[(1R)-1-hydroxyäthyl]-2-penem-3-carbonsäure-Monohydrat-Tetrahydrofuran-Hemisolvat gemäss Anspruch 16.

20. Verfahren gemäss Anspruch 19 zur Herstellung von kristallinem (5R,6S)-2-Aminomethyl-6-[(1R)-1-hydroxyäthyl]-2-penem-3-carbonsäure-Monohydrat-Tetrahydrofuran-Hemisolvat, gekennzeichnet durch die nachfolgenden Gitterabstände (d-Werte über 2,9 Ångström) und relativen Linienintensitäten seines Röntgenpulverdiagramms (Kamera nach Guinier, Strahlungsquelle: Kupfer-$K_{\alpha 1}$):

| d-Werte (Ångström) | relative Intensität |
|---|---|
| 10.2 | sehr stark |
| 9.6 | sehr stark |
| 6.1 | mittel |
| 5.76 | sehr stark |
| 5.11 | mittel |
| 4.82 | stark |
| 4.78 | stark |
| 4.64 | stark |
| 4.41 | sehr schwach |
| 4.07 | mittel |
| 3.98 | stark |
| 3.82 | sehr stark |
| 3.72 | stark |
| 3.58 | sehr stark |
| 3.29 | sehr stark |
| 3.13 | mittel |
| 3.07 | sehr schwach |
| 3.03 | schwach |
| 2.98 | mittel |
| 2.96 | sehr schwach |
| 2.94 | schwach |

21. Verfahren zur Herstellung von pharmazeutischen Präparaten enthaltend (5R,6S)-2-Aminomethyl-6-[(1R)-1-hydroxyäthyl]-2-penem-3-carbonsäure-Monohydrat in kristalliner Form, dadurch gekennzeichnet, dass man kristallines (5R,6S)-2-Aminomethyl-6-[(1R)-1-hydroxyäthyl]-2-penem-3-carbonsäure-Monohydrat mit einem pharmazeutisch annehmbaren Trägerstoff mischt.

FO 7.4/UL/sm*